# EUROPEAN PATENT SPECIFICATION

(11) **EP 2 757 090 B1**
(45) Date of publication and mention of the grant of the patent: **26.04.2017**
(21) Application number: 12831414.3
(22) Date of filing: 06.09.2012
(51) Int. Cl.: C07C 231/02, C07C 233/18, C07C 235/08, C07B 61/00

(54) **METHOD FOR PRODUCING N-ACYLAMINO TRIOL DERIVATIVES**
VERFAHREN ZUR HERSTELLUNG VON N-ACYLAMINOTRIOL DERIVATEN
PROCÉDÉ DE FABRICATION DE DÉRIVÉS N-ACYLAMINO TRIOL

(30) Priority: 13.09.2011 JP 2011199022
(43) Date of publication of application: 23.07.2014
(73) Proprietor: Kao Corporation, Chuo-ku Tokyo 103-8210 (JP)
(72) Inventor: HASHIMOTO, Hiroshi, Haga-gun Tochigi 321-3497 (JP); SUGAI, Yoshiya, Haga-gun Tochigi 321-3497 (JP)
(74) Representative: Vossius & Partner Patentanwälte Rechtsanwälte mbB
(86) International application number: PCT/JP2012/072752
(87) International publication number: WO 2013/038985

(56) References cited:
- EP-A1- 0 736 522
- EP-A2- 0 835 864
- WO-A1-92/06073
- WO-A1-93/20038
- WO-A1-99/58542
- WO-A1-2011/032924
- CA-A1- 2 127 644
- JP-A- H07 145 124
- JP-A- H08 283 218
- JP-A- H10 114 732
- JP-A- S63 216 852
- JP-A- 2011 522 550
- US-A1- 2011 077 302
- WILD R ET AL: "Sphingosine and phytosphingosine from D-threose synthesis of a 4-keto-ceramide", TETRAHEDRON ASYMMETRY, PERGAMON PRESS LTD, OXFORD, GB, vol. 5, no. 11, November 1994 (1994-11), pages 2195-2208, XP026637535, ISSN: 0957-4166, DOI: 10.1016/S0957-4166(00)86295-3 [retrieved on 1994-11-01]
- CHANG Y-T ET AL: "THE SYNTHESIS AND BIOLOGICAL CHARACTERIZATION OF A CERAMIDE LIBRARY", JOURNAL OF THE AMERICAN CHEMICAL SOCIETY, AMERICAN CHEMICAL SOCIETY, vol. 124, no. 9, January 2002 (2002-01), XP008041297, ISSN: 0002-7863, DOI: 10.1021/JA017576O
- FERRERI,C. ET AL.: 'Synthesis of all-trans anandamide: A substrate for fatty acid amide hydrolase with dual effects on rabbit platelet activation' BIOORGANIC & MEDICINAL CHEMISTRY vol. 16, no. 18, 2008, pages 8359 - 8365, XP025427627

## Description

### Field of the Invention

The present invention relates to a method for producing N-acylamino triol from amino triol.

### Background of the Invention

Ceramide 3 which is a typical component having a N-acylamino triol skeleton exists in the stratum corneum and plays an important role in constructing a lipid barrier necessary for moisture retention to keep moisture content. Recently, ceramide or a ceramide functional material having the same function as ceramide is formulated in, for example, cosmetics to provide a moisture retention function from the outside.

Usually, an amide compound is produced by N-acylating an amine compound by using an acid anhydride, acid halide, fatty acid ester, fatty acid and the like in the presence of a basic catalyst. However, in the acylation of amino alcohol having a plurality of alcoholic hydroxyl groups in its molecule, secondary peracylated compounds are easily produced by acylation of a hydroxyl group. It is therefore desired to develop a more selective N-acylation method.

From this point of view, there are a report concerning a method 1) for N-acylating amino alcohol by using a specific mixed acid anhydride (Patent Document 1) and a report concerning a method 2) for N-acylating amino alcohol in a water-containing organic solvent by using an acid halide (Patent Document 2).

The method 1) poses a problem concerning, for example, production of a lot of byproducts, and the method 2) poses, for example, a problem that since the acid halide has very high reactivity and reacts with water to generate harmful hydrogen chloride and the like, special care must be taken to handle the acid halide, which also places high load on equipment and needs a pH control system of reaction.

Also, as the N-acylation reaction of amino alcohol by using a fatty acid ester and a base, there are a report concerning a method 3) in which amino alcohol is reacted in the presence of a basic catalyst such as potassium hydroxide without using any solvent while distilling lower alcohols produced (Patent Document 3), and a report concerning a method 4) in which amino diol and a fatty acid ester are reacted in an alcohol having from 3 to 8 carbon atoms while distilling lower alcohols produced (Patent Document 4).

However, the method 3) poses a problem, for example, that it is unsuitable for the production of a compound such as ceramide 3 (N-acylated phytosphingosine) having a high melting point and peracylated compounds are easily by-produced. Also, the method 4) is a method using amino diol as a raw material and has no description concerning the N-acylation of amino triol largely different in, for example, melting point and solubility.

### Citation List

### Patent Document

Patent Document 1: JP-A-7-505163
Patent Document 2: JP-A-2002-514654
Patent Document 3: JP-A-63-216852
Patent Document 4: Japanese Patent No. 4101320

US 2011/077302 A1 relates to the enzymatic synthesis of sphingolipids and compositions that contain sphingolipids from lysosphingolipids and carbonic esters,

EP 0 736 522 A1, WO 99/58542 A1, WO 93/20038 A1, WILD, R. ET AL: "Sphingosine and phytosphingosine from D-threose synthesis of a 4-keto-ceramide", TETRAHEDRON ASYMMETRY, PERGAMON PRESS LTD, OXFORD, GB, vol. 5, no. 11, 1 November 1994, pages 2195-2208, XP026637535, and CHANG Y-T ET AL: "THE SYNTHESIS AND BIOLOGICAL CHARACTERIZATION OF A CERAMIDE LIBRARY", JOURNAL OF THE AMERICAN CHEMICAL SOCIETY, AMERICAN CHEMICAL SOCIETY, vol. 124, no. 9, 1 January 2002 (2002-01-01), page 1856/1857, XP008041297, disclose methods for preparing compounds of the following formula (1) via acylation of the corresponding polyhydroxyamine derivatives but do not disclose that the triol derivative (2) is reacted with a fatty ester in the presence of a base in ethanol or methanol.

### Summary of the Invention

The present invention relates to a method for producing N-acylamino triol represented by the following formula (1): 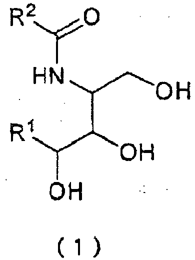 wherein R¹ represents a saturated or unsaturated aliphatic 3 hydrocarbon group having from 8 to 20 carbon atoms and R² represents a saturated or unsaturated aliphatic hydrocarbon group having from 7 to 27 carbon atoms which may have a hydroxyl group, the method comprising reacting amino triol represented by the following formula (2): 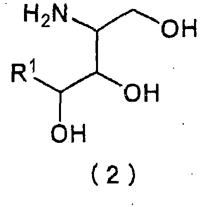 wherein R¹ has the same meaning as above, with a fatty acid ester represented by the following formula (3):

R²COOR³ (3)

wherein R² has the same meaning as above and R³ represents an alkyl group having from 1 to 6 carbon atoms, in ethanol and/or methanol in the presence of a basic catalyst, wherein the amount of the ethanol and/or methanol is 2 to 15 times (mass) the theoretical yield of N-acylamino triol (1).

### Detailed Description of the Invention

The present invention relates to a method for efficiently producing N-acylated amino triol by selectively N-acylating amino triol through industrially advantageous technique.

The inventors of the present invention made studies concerning a simple and efficient method for selective N-acylation of amino triol and as a result, found that N-acylamino triol can be produced with high yield and high purity without any complicated operation by a method of reacting amino triol with a fatty acid ester in the presence of a basic catalyst in ethanol and/or methanol.

The production method of the present invention has the following effects:
1) the reagent used is safe, is easily handled and proceeds with reaction in a mild condition, 2) the method is almost free from the production of peracylated compounds and the like, 3) the purification may be performed only by filtration and no further purification such as recrystallization is required, and 4) the reaction proceeds even in a suspension system in which an object product precipitates. Specifically, according to the method of the present invention, N-acylamino triol typified by ceramide 3 can be produced industrially advantageously.

The method for producing N-acylamino triol according to the present invention is to produce N-acylamino triol (1) by reacting amino triol (2) with a fatty acid ester (3) in the presence of a basic catalyst in ethanol and/or methanol as shown by the following reaction formula. 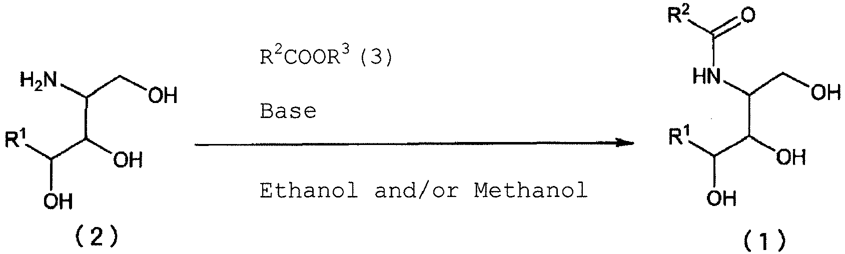 wherein R¹ represents a saturated or unsaturated aliphatic hydrocarbon group having from 8 to 20 carbon atoms, R² represents a saturated or unsaturated aliphatic hydrocarbon group having from 7 to 27 carbon atoms which may have a hydroxyl group, and R³ represents an alkyl group having from 1 to 6 carbon atoms.

As R¹, the saturated or unsaturated aliphatic hydrocarbon group in the formulae (1) and (2) may be either a straight-chain or branched and is preferably saturated or unsaturated aliphatic hydrocarbon group having from 10 to 18 carbon atoms. Also, as the unsaturated aliphatic hydrocarbon group, an alkenyl group is preferable and an alkenyl group having one or two double bonds is more preferable.

More preferable examples of R¹ include straight-chain alkyl groups having from 10 to 18 carbon atoms, for example, a decyl group, undecyl group, dodecyl group, tridecyl group, tetradecyl group, pentadecyl group, hexadecyl group, heptadecyl group, and octadecyl group, among which a tetradecyl group is even more preferable.

As R², the saturated or unsaturated aliphatic hydrocarbon group having from 7 to 27 carbon atoms which may have a hydroxyl group in the formula (1) may either a straight-chain or branched and is preferably saturated or unsaturated aliphatic hydrocarbon group having from 9 to 23 carbon atoms. As the unsaturated aliphatic hydrocarbon group, an alkenyl group is preferable and an alkenyl group having one or two double bonds is more preferable. The number of hydroxyl groups which may be substituted for hydrogen atoms of the saturated or unsaturated aliphatic hydrocarbon group is preferably one or two.

More preferable examples of R² include straight-chain alkyl groups having from 9 to 23 carbon atoms which may have one hydroxyl group, for example, a nonyl group, decyl group, undecyl group, dodecyl group, tridecyl group, tetradecyl group, pentadecyl group, hexadecyl group, heptadecyl group, octadecyl group, nonadecyl group, icosyl group, henicosyl group, 11-hydroxy-heptadecyl group, 1-hydroxy-undecyl group, 1-hydroxy-tridecyl group, 1-hydroxy-pentadecyl group, 1-hydroxy-heptadecyl group, cis-8-heptadecenyl group, and cis,cis-8,11-heptadecadienyl group, among which an undecyl group, pentadecyl group, heptadecyl group, henicosyl group, 11-hydroxy-heptadecyl group, and 1-hydroxy-pentadecyl group are even more preferable.

As R3, the alkyl group having from 1 to 6 carbon atoms in the formula (3) may be straight-chain or branched, and examples thereof include a methyl group, ethyl group, n-propyl group, isopropyl group, and n-butyl group, among which alkyl groups having from 1 to 3 carbon atoms are preferable, and a methyl group, ethyl group, and isopropyl group are more preferable.

Preferable specific examples of the fatty acid ester represented by the formula (3) include methyl nonanoate, ethyl nonanoate, methyl decanoate, ethyl decanoate, methyl undecanoate, ethyl undecanoate, methyl dodecanoate, ethyl dodecanoate, isopropyl dodecanoate, methyl tridecanoate, ethyl tridecanoate, methyl tetradecanoate, ethyl tetradecanoate, methyl pentadecanoate, ethyl pentadecanoate, methylhexadecanoate, ethyl hexadecanoate, isopropylhexadecanoate, methyl heptadecanaote, ethyl heptadecanoate, methyl octadecanoate, ethyl octadecanoate, isopropyl octadecanoate, methyl nonadecanoate, ethyl nonadecanoate, methyl eicosanoate, ethyl eicosanoate, methyl docosanoate, ethyl docosanoate, methyl cis-9-hexadecenoate, ethyl cis-9-hexadecenoate, methyl cis-9-octadecenoate, ethyl cis-9-octadecenoate, methyl cis, cis-9,12-octadecadienoate, ethyl cis, cis-9,12-octadecadienoate, methyl 2-hydroxydodecanoate, ethyl 2-hydroxydodecanoate, methyl 2-hydroxytetradecanoate, ethyl 2-hydroxytetradecanoate, methyl 2-hydroxyhexadecanoate, ethyl 2-hydroxyhexadecanoate, isopropyl 2-hydroxyhexadecanoate, methyl 12-hydroxyoctadecanoate, ethyl 12-hydroxyoctadecanoate, and isopropyl 12-hydroxyoctadecanoate.

It should be noted that compounds represented by the above formulae (1) to (3) may be a single compound or a mixture of compounds in which plural types of R¹ and/or R² exist.

In the method of the present invention, amino triol (2) used as the starting material may be synthesized, for example, by the method described in Liebigs Annalen (1995), (5), 755, Tetrahedron Letters (2003), 44(28), 5281. Specifically, amino triol (2) may be synthesized by utilizing the structure of sugar. Also, amino triol (2) may be produced by the fermentation method using microbial strains which are described in WO/1995/012683 and in the specification of US Patent No. 5958742.

In the acylation reaction in the present invention, ethanol and/or methanol are used as the reaction solvent. It is preferable to use ethanol from the viewpoint of safety, taking it into account that it is used for cosmetic raw material. Also, ethanol and methanol may be used independently or used by appropriately combining them. When an alcohol having 3 or more carbon atoms is used as the reaction solvent, there may be reductions in the yield and purity of N-acylamino triol and deteriorated operability.

The amount of the alcohol solvent used may be appropriately controlled in consideration of the solubility and reactivity of N-acylamino triol which is an object product and amino triol (2) and fatty acid ester (3) which are raw materials, and it is 2 times (mass) or more, more preferably 5 times (mass) or more the theoretical yield of N-acylamino triol (1) which is an object product. Also, the amount of the alcohol solvent is 15 times (mass) or less, more preferably 10 times (mass) or less the theoretical yield of N-acylamino triol (1). Also, the amount of the alcohol solvent is preferably from 5 to 10 times (mass) the theoretical yield of N-acylamino triol (1).

As the base used in this reaction, inorganic bases such as sodium, potassium, potassium hydroxide, sodium hydroxide, sodium carbonate, and potassium carbonate, metal alcoholates such as sodium ethoxide, sodium methoxide, potassium ethoxide, potassium methoxide, sodium iso-propoxide, sodium n-propoxide, sodium n-butoxide, sodium iso-butoxide, potassium iso-propoxide, potassium n-butoxide, potassium iso-butoxide, and potassium t-butoxide, organic bases such as triethylamine and diisopropylethylamine, metal halides such as sodium hydride and potassium hydride, and organic metal bases such as lithium diisopropylamide, lithium bis(trimethylsilyl)amide, sodium bis(trimethylsilyl)amide, and potassium bis(trimethylsilyl)amide may be used without any particular limitation. A metal alcoholate is preferably used, a metal ethylate or methylate is more preferably used, and sodium ethoxide or sodium methoxide is even more preferably used. In this case, it is preferable to use a metal ethylate when the reaction solvent is ethanol or a metal methylate when the reaction solvent is methanol. Either powder products or solution products may be used.

The amount of the base used may be a catalytic amount in usual though it differs depending on the type of base. Specifically, the amount of the base is generally preferably 0.001 mol or more, more preferably 0.01 mol or more based on 1 mol of amino triol (2). Also, the amount of the base is preferably 0.5 mol or less and more preferably 0.3 mol or less. Also, the amount of the base to be used is preferably from 0.001 to 0.5 mol, more preferably from 0.01 to 0.3 mol. When, for example, a metal alcoholate such as sodium alkoxide is used, an amount of about from 0.05 to 0.2 mol of the base used is enough for the requirement.

The amount of the fatty acid ester (3) used is generally preferably 1 mol or more, more preferably 1.1 mol or more based on 1 mol of amino triol (2). Then, the amount of the fatty acid ester (3) is preferably 3 mol or less, 2.0 mol or less. The amount of the fatty acid ester (3) is preferably from 1 to 3 mol, more preferably from 1.1 to 2.0 mol.

Although no particular limitation is imposed on the order of amino triol (2), fatty acid ester (3), and base poured into a reaction system, it is preferable, for example, to add the base after amino triol (2) and fatty acid ester (3) are dissolved in an alcohol solvent in advance from the viewpoint of increasing the purity of an object product. This reason is as follows. The reaction proceeds at once when the base is added and the precipitation of an object product starts depending on the structure and condition. If this reaction proceeds before the raw material is perfectly dissolved in a solvent, non-dissolved raw material is incorporated into the precipitate and there is therefore a fear as to reduced purity.

The reaction temperature is generally preferably 20°C or more, more preferably 40°C or more. Then, the reaction temperature is preferably 79°C or less, more preferably 70°C or less. Also, the reaction temperature is preferably from 20°C to 79°C and more preferably from 40°C to 70°C.

The reaction time is generally preferably 6 hours or more, more preferably 12 hours or more. Then, the reaction time is preferably 48 hours or less, more preferably 36 hours or less. Also, the reaction time is preferably from 6 to 48 hours, more preferably from 12 to 36 hours.

Also, even in the case where, for example, the reaction is undergone at low temperatures and therefore, object N-acylamino triol (1) precipitates, entailing a suspension reaction system (inhomogeneous system), the reaction progresses more satisfactorily even at low temperatures than in the case where the reaction system becomes an inhomogeneous system (see Examples). Moreover, the fear as to the generation of per-acylated compounds can be reduced, resulting in smaller load on equipment.

This reaction may be undergone in an inert gas atmosphere, for example, an argon or nitrogen atmosphere according to the need.

When a methyl ester or ethyl ester is used as the fatty acid ester (3) and a metal ethylate or metal methylate is used as the base in the present invention, byproducts generated in the acylation reaction are ethanol or methanol which is the same as the solvent. It is therefore unnecessary to distill these byproducts out of the system. Therefore, in the present invention, it is unnecessary to undergo reaction under reduced pressure or by introducing inert gas to remove these byproducts out of the system.

In the present invention, the isolation of object N-acylamino triol (1) may be carried out by adding a solvent according to the need and by cooling the reaction solution according to the need, followed by filtration, washing, and drying. Since almost no byproduct is produced as mentioned above, a high purity product can be produced without any necessity of purification such as recrystallization and various chromatographies which are regularly used in organic synthetic chemistries.

With regard to the aforementioned embodiments, the following aspects are disclosed in the present invention. 1) A method for producing N-acylamino triol represented by the following formula (1): 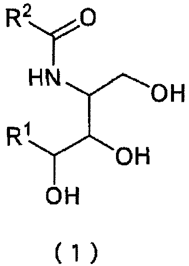 wherein R¹ represents a saturated or unsaturated aliphatic hydrocarbon group having from 8 to 20 carbon atoms and R² represents a saturated or unsaturated aliphatic hydrocarbon group having from 7 to 27 carbon atoms which may have a hydroxyl group, the method comprising reacting amino triol represented by the following formula (2): 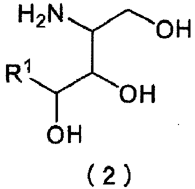 wherein R¹ has the same meaning as above, with a fatty acid ester represented by the following formula (3):

R²COOR³ (3)

wherein R² has the same meaning as above and R³ represents an alkyl group having from 1 to 6 carbon atoms, in ethanol and/or methanol (2 to 15 times (mass) the theoretical yield of acetylated triol) in the presence of a basic catalyst.
2) The method of the above 1), wherein the reaction is undergone at from 20 to 79°C.
3) The method of the above 1) or 2), wherein the reaction is undergone while precipitating an object product.
4) The method of any one of the above 1) to 3), wherein R³ is a methyl group or an ethyl group.
5) The method of any one of the above 1) to 4), wherein the basic catalyst is a metal ethylate or a metal methylate.
6) The method of any one of the above 1) to 5), wherein R¹ is a tetradecyl group.
7) The method of any one of the above 1) to 6), wherein the base is added to undergo reaction after amino triol and fatty acid ester are dissolved in an alcohol solvent in advance.

### Examples

The present invention will be explained in more detail by way of examples.

In the following examples, various data was measured by the following analytical instruments and conditions.
1) Melting point: Melting Point Measuring Instrument 535 (manufactured by BUCHI Labortechnik AG)
2) Infrared absorption spectrum: SPECTRUM ONE (B) (manufactured by PERKIN ELMER, Inc.)
3) Nuclear magnetic resonance spectrum: Avace 500 (manufactured by BRUKER Corporation)
4) Purity calculation: Analyzing TLC band intensity by Lane & spot analyzer 6.0 (manufactured by ATTO Corporation)

### Example 1 Synthesis of N-docosanoyl-phytosphingosine

1.68 g of methyl behenate (manufactured by Sigma Aldrich Co. , LLC.), 1.00 g of phytosphingosine (manufactured by Cosmoferm B.V.), and 16 mL of ethanol (manufactured by Kanto Chemical Co. , Inc.) were added in a 30-mL eggplant flask and the mixture was stirred in a 55°C hot water bath for 15 min. After it was confirmed that the reaction system was homogeneous, 124 µL of a 21% sodium ethoxide-ethanol solution (manufactured by Sigma Aldrich Co., LLC.) was added to the mixture. After 2 hours, a precipitate was confirmed and the mixture was continuously stirred during a whole day and night. After the resulting reaction mixture was cooled to 0°C, the precipitate was filtered and washed to obtain 1.90 g of N-docosanoyl-phytosphingosine in the form of a white solid in a yield of 94.1% (purity > 98%).

Melting point 120-122°C
IR (ATR, cm⁻¹) 3326, 2913, 2849, 1613, 1556, 1467, 1075, 720
MS (positive): [M + H]⁺ = 640.8, [M + Na]⁺ = 662.7
¹H-NMR (deuterated pyridine, 500MHz, δ) 0.85 (3H, t, J = 7.1Hz), 0.85 (3H, t, J = 7.1Hz), 1.16-1.48 (58H, br), 1.69 (1H, m), 1.82 (2H, m), 1.94(2H, m), 2.24 (1H, m), 2.45 (2H, t, J = 7.4Hz), 4.29 (1H, m), 4.40 (1H, ddd, J = 6.2, 6.2, 5.2Hz), 4.49 (2H, m), 5.10 (1H, m), 6.19 (1H, d, J = 6.9Hz), 6.53 (1H, d, J = 6.9Hz), 6.62 (1H, t, J= 5.0Hz), 8.51 (1H, d, J = 8.3Hz)

### Example 2 Synthesis of N-octadecanoyl-phytosphingosine

1.47 g of ethyl stearate (manufactured by Kanto Chemical Co., Inc.), 1.00 g of phytosphingosine, and 13.2 mL of ethanol were added in a 30-mL eggplant flask and the mixture was stirred in a 55°C hot water bath for 15 min. After it was confirmed that the reaction system was homogeneous, 123 µL of a 21% sodium ethoxide-ethanol solution was added to the mixture. After 2 hours, a precipitate was confirmed and the mixture was continuously stirred during a whole day and night.

After the resulting reaction mixture was cooled to 30°C, the precipitate was filtered and washed to obtain 1.61 g of N-octadecanoyl-phytosphingosine in the form of a white solid in a yield of 87.5% (purity > 98%).

Melting point 125- 126°C
IR (ATR, cm⁻¹) 3338, 2954, 2915, 2950, 1611, 1541, 1472, 1075, 716
MS (positive): [M + H]⁺ = 584.6, [M + Na]⁺ = 606.5
¹H-NMR (deuterated pyridine, 500MHz, δ) 0.85 (3H, t, J = 6.9Hz), 0.85 (3H, t, J = 6.9Hz), 1.16-1.49 (50H, br), 1.69 (1H, m), 1.82 (2H, m), 1.94(2H, m), 2.24 (1H, m), 2.45 (2H, t, J = 7.5Hz), 4.29 (1H, m), 4.40 (1H, ddd, J = 5.9, 5.9, 5.5Hz), 4.49 (2H, m), 5.10 (1H, m), 6.19 (1H, d, J = 6.8Hz), 6.53 (1H, d, J= 6.6Hz), 6.62 (1H, t, J= 5.0Hz), 8.51 (1H, d, J = 8.3Hz)

### Example 3 Synthesis of N-octadecanoyl-phytosphingosine

1.47 g of ethyl stearate (manufactured by Kanto Chemical Co., Inc.), 1.00 g of phytosphingosine, and 13.2 mL of ethanol were added in a 30-mL eggplant flask and the mixture was stirred in a 75°C hot water bath for 15 min. After it was confirmed that the reaction system was homogeneous, 123 µL of a 21% sodium ethoxide-ethanol solution was added to the mixture, which was then continuously stirred during a whole day and night. After the resulting reaction mixture was cooled to 30°C, the precipitate was filtered and washed to obtain 1.57 g of N-octadecanoyl-phytosphingosine in the form of a white solid in a yield of 86.3% (purity > 98%).

Melting point 125-126°C
IR (ATR, cm⁻¹) 3338, 2954, 2915, 2950, 1611, 1541, 1472, 1075, 716
MS (positive): [M + H]⁺ = 584.6, [M + Na]⁺ = 606.5
¹H-NMR (deuterated pyridine, 500MHz, δ) 0.85 (3H, t, J = 6.9Hz), 0.85 (3H, t, J = 6.9Hz), 1.16-1.49 (50H, br), 1.69 (1H, m), 1.82 (2H, m), 1.94(2H, m), 2.24 (1H, m), 2.45 (2H, t, J = 7.5Hz), 4.29 (1H, m), 4.40 (1H, ddd, J=5.9, 5.9, 5.5Hz), 4.49 (2H, m), 5.10 (1H, m), 6.19 (1H, d, J = 6.8Hz), 6.53 (1H, d, J = 6.6Hz), 6.62 (1H, t, J = 5.0Hz), 8.51 (1H, d, J = 8.3Hz)

### Example 4 Synthesis of N-hexadecanoyl-phytosphingosine

1.11 g of ethyl palmitate (manufactured by Kanto Chemical Co., Inc.), 1.00 g of phytosphingosine, and 14 mL of methanol were added in a 30-mL eggplant flask and the mixture was stirred in a 55°C hot water bath for 15 min. After it was confirmed that the reaction system was homogeneous, 315 µL of a 1 mol/L sodium methoxide-methanol solution (manufactured by Kanto Chemical Co., Inc.) was added to the mixture. After 2 hours, a precipitate was confirmed and the mixture was continuously stirred during a whole day and night. After the resulting reaction mixture was cooled to 30°C, the precipitate was filtered and washed to obtain 1.55 g of N-hexadecanoyl-phytosphingosine in the form of a white solid in a yield of 88.6% (purity > 98%).

Melting point 114- 117°C
IR (ATR, cm⁻¹) 3336, 2917, 2849, 1611, 1542, 1468, 1076, 717
MS (positive): [M + H]⁺ = 556.7, [M + Na]⁺ = 584.7
¹H-NMR (deuterated pyridine, 500MHz, δ) 0.85 (3H, t, J = 7.0Hz), 0.85 (3H, t, J = 7.0Hz), 1.16-1.49 (46H, br), 1.69 (1H, m), 1.82 (2H, m), 1.94(2H, m), 2.24 (1H, m), 2.45 (2H, t, J = 7.6Hz), 4.29 (1H, m), 4.40 (1H, ddd, J = 5.9, 5.9, 5.5Hz), 4.49 (2H, m), 5.10 (1H, m), 6.19 (1H, d, J = 6.7Hz), 6.53 (1H, d, J = 6.2Hz), 6.62 (1H, t, J = 5.4Hz), 8.52 (1H, d, J = 8.5Hz)

### Example 5 Synthesis of N-hexadecanoyl-phytosphingosine

1.41 g of isopropyl palmitate (manufactured by Wako Pure Chemical Industries Ltd.), 1.00 g of phytosphingosine, and 14 mL of ethanol were added in a 30-mL eggplant flask and the mixture was stirred in a 55°C hot water bath for 15 min. After it was confirmed that the reaction system was homogeneous, 17.7 mg of potassium hydroxide (manufactured by Kanto Chemical Co. , Inc.) was added. After 2 hours, a precipitate was confirmed and the mixture was continuously stirred during a whole day and night. After the resulting reaction mixture was cooled to 30°C, the precipitate was filtered and washed to obtain 1.45 g of N-hexadecanoyl-phytosphingosine in the form of a white solid in a yield of 82.9% (purity: 95%).

Melting point 114-117°C
IR (ATR, cm⁻¹) 3336, 2917, 2849, 1611, 1542, 1468, 1076, 717
MS (positive): [M + H]⁺ = 556.7, [M + Na]⁺ = 584.7
¹H-NMR (deuterated pyridine, 500MHz, δ) 0.85 (3H, t, J = 7.0Hz), 0.85 (3H, t, J = 7.0Hz), 1.16-1.49 (46H, br), 1.69 (1H, m), 1.82 (2H, m), 1.94(2H, m), 2.24 (1H, m), 2.45 (2H, t, J = 7.6Hz), 4.29 (1H, m), 4.40 (1H, ddd, J = 5.9, 5.9, 5.5Hz), 4.49 (2H, m), 5.10 (1H, m), 6.19 (1H, d, J = 6.7Hz), 6.53 (1H, d, J = 6.2Hz), 6.62 (1H, t, J = 5.4Hz), 8.52 (1H, d, J = 8.5Hz)

### Example 6 Synthesis of N-dodecanoyl-phytosphingosine

1.31 g of methyl dodecanoate (manufactured by Wako Pure Chemical Industries Ltd.), 1.50 g of phytosphingosine, and 16 mL of ethanol were added in a 30-mL eggplant flask and the mixture was stirred in a 75°C hot water bath for 15 min. After it was confirmed that the reaction system was homogeneous, 123 µL of a 21% sodium ethoxide-ethanol solution was added. The mixture was continuously stirred during a whole day and night. After the resulting reaction mixture was cooled to ambient temperature, the precipitate was filtered and washed to obtain 1.96 g of N-dodecanoyl-phytosphingosine in the form of a white solid in a yield of 83.5% (purity > 98%).

Melting point 112-114°C
IR (ATR, cm⁻¹) 3325, 2917, 2850, 1612, 1556, 1467, 1076, 721
MS (positive): [M + H]⁺ = 500.9, [M + Na]⁺ = 522.6
¹H-NMR (deuterated pyridine, 500MHz, δ) 0.85 (3H, t, J = 7.0Hz), 0.85 (3H, t, J = 7.0Hz), 1.16-1.49 (46H, br), 1.69 (1H, m), 1.82 (2H, m), 1.94(2H, m), 2.24 (1H, m), 2.45 (2H, t, J = 7.4Hz), 4.29 (1H, m), 4.40 (1H, ddd, J = 5. 6, 5. 6, 5.5Hz), 4.49 (2H, m), 5.10 (1H, m), 6.19 (1H, d, J = 6.6Hz), 6.53 (1H, d, J = 6.2Hz), 6. 62 (1H, t, J = 5.2Hz), 8.52 (1H, d, J = 8.5Hz)

### Example 7 Synthesis of N-(12-hydroxyoctadecanoyl)-phytosphingosine

1.39 g of methyl 12-hydroxyoctadecanoate (manufactured by Sigma Aldrich Co., LLC.), 1.00 g of phytosphingosine, and 13.2 mL of methanol were added in a 30-mL eggplant flask and the mixture was stirred in a 55°C hot water bath for 15 min. After it was confirmed that the reaction system was homogeneous, 315 µL of a 1 mol/L sodium methoxide-methanol solution was added. After 3 hours, a precipitate was confirmed and the mixture was continuously stirred during a whole day and night. After the resulting reaction mixture was cooled to 30°C, the precipitate was filtered and washed to obtain 1.68 g of N-(12-hydroxyoctadecanoyl)-phytosphingosine as a diastereo mixture in the form of a white solid in a yield of 89.2% (purity: 96%).

Melting point 137-139°C
IR (ATR, cm⁻¹) 3359, 2917, 2850, 1618, 1553, 1469, 1076, 1041, 722
MS (positive): [M + H]⁺ = 600.7, [M + Na]⁺ = 622.6

### Example 8 Synthesis of

### N-(2-hydroxyhexadecanoyl)-phytosphingosine

1.26 g of methyl 2-hydroxypalmitate (manufactured by TCI), 1.00 g of phytosphingosine, and 13.2 mL of methanol were added in a 30-mL eggplant flask and the mixture was stirred in a 55°C hot water bath for 15 min. After it was confirmed that the reaction system was homogeneous, 315 µL of a 1 mol/L sodium methoxide-methanol solution was added. The mixture was continuously stirred during a whole day and night. After the resulting reaction mixture was cooled to ambient temperature, the precipitate was filtered and washed to obtain 1.60 g of N-(2-hydroxyhexadecanoyl)-phytosphingosine as a diastereo mixture in the form of a white solid in a yield of 88.8% (purity: 98%).

Melting point 124-129°C
IR (ATR, cm⁻¹) 3306, 2917, 2849, 1632, 1620, 1537, 1467, 1072, 720
MS (positive): [M + H]⁺ = 572.7, [M + Na]⁺ = 594.7

### Comparative Example 1 Synthesis of

### N-octadecanoyl-phytosphingosine

N-octadecanoyl-phytosphingosine was synthesized as follows according to the method described in JP-A-63-216852 (aforementioned Patent Document 3).

5.00 g of phytosphingosine was poured into a 100 mL two-neck egg plant flask and melted by heating with stirring in a nitrogen atmosphere. It was necessary to heat the mixture up to 145°C to melt phytosphingosine completely. When 88 mg of potassium hydroxide was added and the mixture was stirred under reduced pressure of 10 kpa, a precipitate was produced, so that the stirring was stopped, and therefore, the mixture was further heated up to 155°C to melt the mixture. 4.98 g of methyl stearate (manufactured by Kanto Chemical Co., Inc.) was added dropwise to the mixture by using a dropping funnel and the resulting mixture was stirred under a reduced pressure of 10 kpa for 1.5 hours. Complicated products were confirmed on TLC. The resulting mixture was crystallized twice from acetone and ethanol. However, an object product could not be obtained with high purity. The amount of the product was 4.01 g and yield was 43.7% (purity: 67%).

### Comparative Example 2 Synthesis of

### N-octadecanoyl-phytosphingosine

N-octadecanoyl-phytosphingosine was synthesized as follows according to the method described in JP-4101320 (aforementioned Patent Document 4).

1. 59 g of phytosphingosine, 50 mg of potassium hydroxide, and 15 mL of butanol (manufactured by Kanto Chemical Co., Inc.) were poured into a 100 mL two-neck egg plant flask and stirred at 90°C for 30 min. A solution containing 2.24 g of methyl stearate and 5 mL of butanol was added dropwise to the mixture by using a dropping funnel while introducing nitrogen and the mixture was stirred at 90°C for 2 hours. Although the raw material remained, products other than the object product were also confirmed on TLC. The obtained mixture was cooled to -10°C and the precipitate was filtered and washed. However, the object product could not be obtained with high purity. The amount of the product was 2.00 g and yield was 68.5% (purity: 80%).

## Claims

1. A method for producing N-acylamino triol represented by the following formula (1): 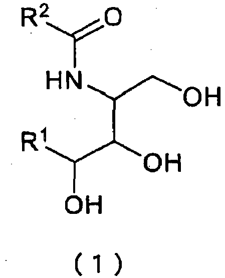 wherein R¹ represents a saturated or unsaturated aliphatic hydrocarbon group having from 8 to 20 carbon atoms and R² represents a saturated or unsaturated aliphatic hydrocarbon group having from 7 to 27 carbon atoms which may have a hydroxyl group, the method comprising reacting amino triol represented by the following formula (2): 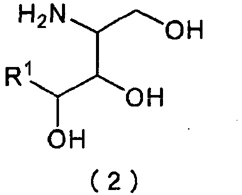 wherein R¹ has the same meaning as above, with a fatty acid ester represented by the following formula (3):
R²COOR³ (3)
wherein R² has the same meaning as above and R³ represents an alkyl group having from 1 to 6 carbon atoms, in ethanol and/or methanol in the presence of a basic catalyst, wherein the amount of the ethanol and/or methanol is 2 to 15 times (mass) the theoretical yield of N-acylamino triol (1).

2. The method according to claim 1, wherein the reaction is undergone at 20°C or more and 79°C or less.

3. The method according to claim 1 or 2, wherein the reaction is undergone while precipitating an object product.

4. The method according to any one of claims 1 to 3, wherein R¹ represents a saturated or unsaturated aliphatic hydrocarbon group having from 10 to 18 carbon atoms.

5. The method according to any one of claims 1 to 4, wherein R² represents a saturated or unsaturated aliphatic hydrocarbon group having from 9 to 23 carbon atoms.

6. The method according to any one of claims 1 to 5, wherein R¹ is a tetradecyl group.

7. The method according to any one of claims 1 to 6, wherein R³ is a methyl group or an ethyl group.

8. The method according to any one of claims 1 to 7, wherein the basic catalyst is a metal ethylate or a metal methylate.

9. The method according to claim 1, wherein an amount of the ethanol and/or methanol is 5 times (mass) or more and 10 times or less the theoretical yield of N-acylamino triol (1) .

10. The method according to any one of claims 1 to 9, wherein an amount of the basic catalyst is 0.01 mol or more and 0.3 mol or less based on 1 mol of amino triol (2).

11. The method according to any one of claims 1 to 10, wherein an amount of the fatty acid ester (3) is 1.1 mol or more and 2.0 mol or less based on 1 mol of amino triol (2).

12. The method according to any one of claims 1 to 11, wherein a reaction time is 12 hours or more and 36 hours or less.

13. The method according to any one of claims 1 to 12, wherein the base is added to undergo reaction after the amino triol and the fatty acid ester are dissolved in the alcohol solvent in advance.

## Patentansprüche

1. Ein Verfahren zur Herstellung von N-Acylaminotriol, dargestellt durch die folgende Formel (1): 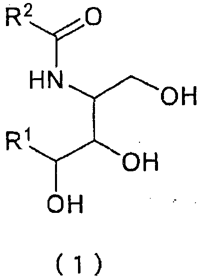 wobei R¹ eine gesättigte oder ungesättigte aliphatische Kohlenwasserstoffgruppe mit 8 bis 20 Kohlenstoffatomen darstellt und R² eine gesättigte oder ungesättigte aliphatische Kohlenwasserstoffgruppe mit 7 bis 27 Kohlenstoffatomen darstellt, welche eine Hydroxylgruppe aufweisen kann, wobei das Verfahren das Umsetzen von Aminotriol, dargestellt durch die folgende Formel (2): 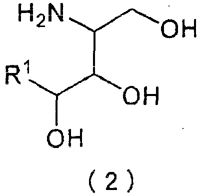 wobei R¹ die gleiche Bedeutung wie oben hat, mit einem Fettsäureester, dargestellt durch die folgende Formel (3):
R²COOR³ (3)
wobei R² die gleiche Bedeutung wie oben hat und R³ eine Alkylgruppe mit 1 bis 6 Kohlenstoffatomen darstellt, in Ethanol und/oder Methanol in Gegenwart eines basischen Katalysators umfasst, wobei die Menge des Ethanols und/oder Methanols das 2- bis 15-fache (Masse) der theoretischen Ausbeute von N-Acylaminotriol (1) beträgt.

2. Das Verfahren gemäß Anspruch 1, wobei die Umsetzung bei 20°C oder mehr und 79°C oder weniger stattfindet.

3. Das Verfahren gemäß Anspruch 1 oder 2, wobei die Umsetzung unter Ausfällen eines gewünschten Produktes stattfindet.

4. Das Verfahren gemäß einem der Ansprüche 1 bis 3, wobei R¹ eine gesättigte oder ungesättigte aliphatische Kohlenwasserstoffgruppe mit 10 bis 18 Kohlenstoffatomen darstellt.

5. Das Verfahren gemäß einem der Ansprüche 1 bis 4, wobei R² eine gesättigte oder ungesättigte aliphatische Kohlenwasserstoffgruppe mit 9 bis 23 Kohlenstoffatomen darstellt.

6. Das Verfahren gemäß einem der Ansprüche 1 bis 5, wobei R¹ eine Tetradecylgruppe ist.

7. Das Verfahren gemäß einem der Ansprüche 1 bis 6, wobei R³ eine Methylgruppe oder eine Ethylgruppe ist.

8. Das Verfahren gemäß einem der Ansprüche 1 bis 7, wobei der basische Katalysator ein Metallethylat oder ein Metallmethylat ist.

9. Das Verfahren gemäß Anspruch 1, wobei eine Menge des Ethanols und/oder Methanols das 5-fache (Masse) oder mehr und das 10-fache oder weniger der theoretischen Ausbeute von N-Acylaminotriol (1) beträgt.

10. Das Verfahren gemäß einem der Ansprüche 1 bis 9, wobei eine Menge des basischen Katalysators 0,01 Mol oder mehr und 0,3 Mol oder weniger beträgt, bezogen auf 1 Mol Aminotriol (2).

11. Das Verfahren gemäß einem der Ansprüche 1 bis 10, wobei eine Menge des Fettsäureesters (3) 1,1 Mol oder mehr und 2,0 Mol oder weniger beträgt, bezogen auf 1 Mol Aminotriol (2).

12. Das Verfahren gemäß einem der Ansprüche 1 bis 11, wobei eine Reaktionszeit 12 Stunden oder mehr und 36 Stunden oder weniger beträgt.

13. Das Verfahren gemäß einem der Ansprüche 1 bis 12, wobei die Base zugegeben wird um zu reagieren, nachdem das Aminotriol und der Fettsäureester vorher in dem Alkohollösungsmittel gelöst wurden.

## Revendications

1. Procédé de production d'un N-acylaminotriol, représenté par la formule (1) ci-après : 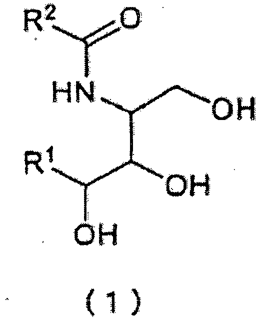 dans laquelle R¹ représente un groupe hydrocarboné aliphatique saturé ou insaturé ayant 8 à 20 atomes de carbone, et R² représente un groupe hydrocarboné aliphatique saturé ou insaturé ayant de 7 à 27 atomes de carbone, qui peut avoir un groupe hydroxyle, le procédé comprenant la réaction d'un aminotriol représenté par la formule (2) ci-après : 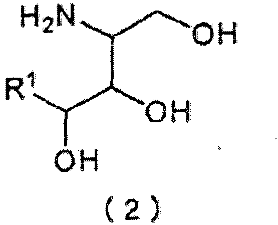 dans laquelle R¹ a la même signification que ci-dessus, avec un ester d'acide gras représenté par la formule (3) ci-après :
R²COOR³ (3)
dans laquelle R² a la même signification que ci-dessus, et R³ représente un groupe alkyle ayant 1 à 6 atomes de carbone, dans de l'éthanol et/ou du méthanol en présence d'un catalyseur basique, la quantité de l'éthanol et/ou du méthanol étant 2 à 15 fois (en masse) le rendement théorique en N-acylaminotriol (1).

2. Procédé selon la revendication 1, dans lequel la réaction est mise en oeuvre à 20°C ou plus et à 79°C ou moins.

3. Procédé selon la revendication 1 ou 2, dans lequel la réaction est mise en oeuvre avec précipitation d'un produit objectif.

4. Procédé selon l'une quelconque des revendications 1 à 3, dans lequel R¹ représente un groupe hydrocarboné aliphatique saturé ou insaturé ayant de 10 à 18 atomes de carbone.

5. Procédé selon l'une quelconque des revendications 1 à 4, dans lequel R² représente un groupe hydrocarboné aliphatique saturé ou insaturé ayant de 9 à 23 atomes de carbone.

6. Procédé selon l'une quelconque des revendications 1 à 5, dans lequel R¹ est un groupe tétradécyle.

7. Procédé selon l'une quelconque des revendications 1 à 6, dans lequel R³ est un groupe méthyle ou un groupe éthyle.

8. Procédé selon l'une quelconque des revendications 1 à 7, dans lequel le catalyseur basique est un éthylate métallique ou un méthylate métallique.

9. Procédé selon la revendication 1, dans lequel la quantité de l'éthanol et/ou du méthanol est 5 fois (en masse) ou plus et 10 fois ou moins le rendement théorique en N-acylaminotriol (1).

10. Procédé selon l'une quelconque des revendications 1 à 9, dans lequel la quantité du catalyseur basique est de 0,01 mole ou plus et de 0,3 mole ou moins par mole de l'aminotriol (2).

11. Procédé selon l'une quelconque des revendications 1 à 10, dans lequel la quantité de l'ester d'acide gras (3) est de 1,1 mole ou plus et de 2,0 moles ou moins par mole de l'aminotriol (2).

12. Procédé selon l'une quelconque des revendications 1 à 11, dans lequel le temps de réaction est de 12 heures ou plus et de 36 heures ou moins.

13. Procédé selon l'une quelconque des revendications 1 à 12, dans lequel la base est ajoutée pour mettre en oeuvre une réaction après que l'aminotriol et l'ester d'acide gras se sont au préalable dissous dans le solvant alcool.
